# EUROPEAN PATENT SPECIFICATION

(11) **EP 2 800 514 B1**
(45) Date of publication and mention of the grant of the patent: **14.04.2021**
(21) Application number: 13703138.1
(22) Date of filing: 03.01.2013
(51) Int. Cl.: A61B 5/12

(54) **APPARATUS FOR CLINICAL-AUDIOMETRIC INVESTIGATION**
VORRICHTUNG FÜR KLINISCH-AUDIOMETRISCHE UNTERSUCHUNG
APPAREIL POUR EXAMEN CLINIQUE AUDIOMÉTRIQUE

(30) Priority: 03.01.2012 IT PD20120002
(43) Date of publication of application: 12.11.2014
(73) Proprietor: Neuranix S.r.l., 80124 Napoli (IT)
(72) Inventor: SACCHI, Giorgio, I-44124 Ferrara (IT); STANZIAL, Domenico, I-44123 Ferrara (IT)
(74) Representative: Modiano, Micaela Nadia
(86) International application number: PCT/IB2013/050060
(87) International publication number: WO 2013/102867

(56) References cited:
- DE-A1- 10 131 823
- US-A- 4 688 582
- HIIPAKKA M ET AL: "Estimating pressure at eardrum with pressure-velocity measurement from ear canal entrance", APPLICATIONS OF SIGNAL PROCESSING TO AUDIO AND ACOUSTICS, 2009. WASPAA '09. IEEE WORKSHOP ON, IEEE, PISCATAWAY, NJ, USA, 18 October 2009 (2009-10-18), pages 289-292, XP031575136, ISBN: 978-1-4244-3678-1
- STANZIAL DOMENICO ET AL: "Calibration of pressure-velocity probes using a progressive plane wave reference field and comparison with nominal calibration filters", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 129, no. 6, 1 June 2011 (2011-06-01), pages 3745-3755, XP012136601, ISSN: 0001-4966, DOI: 10.1121/1.3589253

## Description

The present invention relates to an apparatus for clinical-audiometric investigation.

An apparatus for audiometric investigations according to the state of the art is for example described in DE10131823A1.

In recent decades apparatuses have been developed for clinical-audiometric tests with a view to improving the investigation process and the detection of diseases of the tympanic membrane of the middle ear.

In general, the energy of any acoustic disturbance is completely described by the acoustic pressure field and acoustic particle velocity, i.e. the speed of vibration of the air caused by the small variations of atmospheric pressure owing to the presence of the sound. The quantity of acoustic energy transmitted and reflected can in fact be estimated with the measurement of acoustic impedance, which can be determined as the ratio between the acoustic pressure and the speed of vibration of the particles at a point.

In the specific case of the auditory canal, given its particular shape structure, the air is restricted to oscillating almost only along the axis of the canal and this makes it possible, in audiometric measurements, to approximate the velocity vector with a monodimensional quantity.

The acoustics of the auditory canal are therefore described in terms of pressure and velocity measured in the direction of the axis of the auditory canal.

However, currently existing technology in the field of acoustic impedenziometry is based on measurement of the acoustic pressure field alone: the systems in use employ a single microphonic sensor for the measurement of the pressure at a point, from which the acoustic particle velocity at that point is implemented.

Moreover, at present impedenziometers take advantage of a calibration of the hybrid type, i.e. mixed electrical and acoustic in nature, in order to approximately and indirectly determine the velocity signal which is calibrated by comparison with the pressure signal for known volumes of air.

The systems thus process a single signal corresponding to the response under pressure for single exciting frequencies.

Insofar as the implementation of the velocity is accurate, such systems exhibit the drawback of not being precise in the audiometric investigation process.

The aim of the present invention is to provide an apparatus for clinical-audiometric investigation which is capable of measuring energy acoustic values precisely and reliably.

Within this aim, an object of the invention is to provide an apparatus for clinical-audiometric investigation which is a valid tool for detecting diseases of the tympanic membrane of the middle ear.

This aim and these and other objects which will become more apparent hereinafter are all achieved by an apparatus according to the invention, which is defined in the claims.

Further characteristics and advantages will become more apparent from the detailed description that follows of a preferred, but not exclusive, embodiment of the apparatus for clinical-audiometric investigation according to the invention, which is illustrated for the purposes of non-limiting example in the accompanying drawings wherein:
Figure 1 is a perspective view of the apparatus according to the invention;
Figure 2 is an enlargement of the processing and control device, with some of its elements shown schematically and prominently.

With reference to the figures, the apparatus according to the invention, which is generally designated with the reference numeral 10, has a box-like body 11, which contains an audio source 12 of an acoustic signal A and a detector 13 of the return signal B from a measurement environment 14, which in the case shown consists of an external auditory canal.

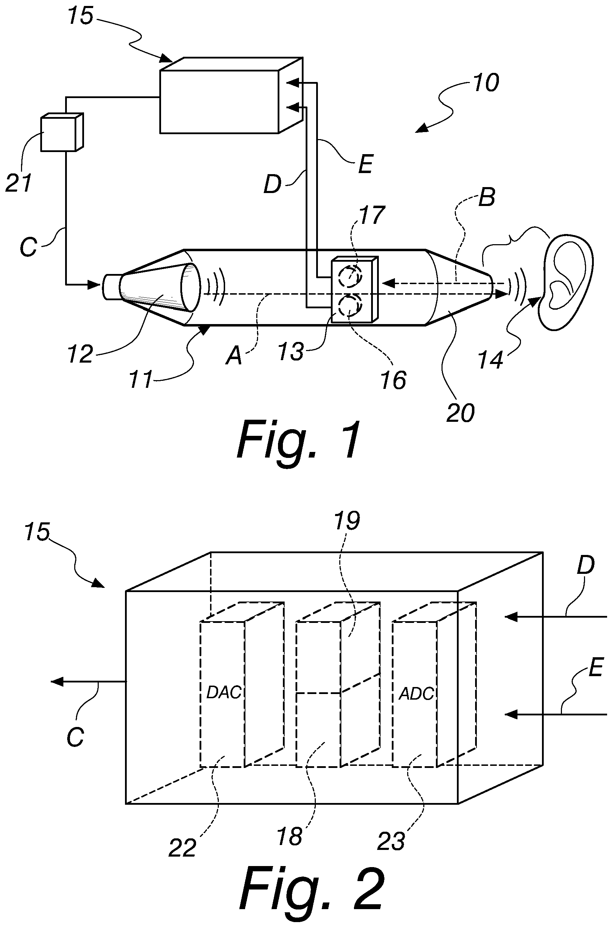

The box-like body 11 is associated with a processing and control device 15, which is shown in greater detail in Figure 2.

The return signal detector 13 advantageously comprises a velocity sensor 16 and a pressure sensor 17 which measure the acoustic velocity and pressure signals at the same point.

In particular, the velocity sensor 16 is constituted by an acoustic anemometer and the pressure sensor 17 is constituted by a microphone.

The box-like body 11 conveniently also comprises an adapter 20 for coupling to the measurement environment 14, i.e. to the external auditory canal.

In particular, the box-like body 11 is substantially cylindrical and hollow and has the audio source 12 at one end and terminates at the other end in the adapter 20 which is frustum-shaped.

Advantageously, the processing and control device 15 comprises a first calculation section 18 and a second calculation section 19, which are assigned respectively to calculating the acoustic immittance and the energy analysis of the return signal B.

The apparatus 10 also comprises an amplifier 21 of the signal C in output from the processing and control device 15 and directed toward the audio source 12.

Conveniently, the processing and control device 15 is provided with a digital-to-analog converter 22 of the output signal C and with an analog-to-digital converter 23 of a first input signal D originating from the velocity sensor 16 and of a second input signal E originating from the pressure sensor 17.

The operation of the apparatus is as follows.

An operator generates, by way of a data management program, a wide band digital signal which is converted to analog by way of the digital-to-analog converter 22, amplified, and sent, as an output signal C, to the audio source 12, which generates the acoustic signal A that excites the auditory canal of the ear (or in general the measurement environment 14).

The reflected acoustic energy generates a return signal B to the detector 13 which measures, at the same point, respectively by means of the acoustic anemometer (velocity sensor 16) and the microphone (pressure sensor 17), the velocity and pressure fields.

The first input signal D and the second input signal E to the processing and control device 15, which are transduced by the detector 13, are digitalized by the analog-to-digital converter 23 and stored.

The data thus collected are ready for the subsequent processing, which occurs by way of two mutually independent calculation sections.

The first calculation section 18 calculates the acoustic immittance, i.e. it calculates the values that are traditionally monitored in acoustic impedenziometry.

The second calculation section 19 calculates an energy analysis of the two signals.

In particular this analysis consists in determining the conductance susceptance of the energy and the distribution of their normalized values is represented in the desired frequency band.

More specifically, from the frequency band representation of the normalized conductance the value of the energy absorbance can be found, which explicitly indicates the percentage of acoustic energy absorbed by the ossicular-tympanum system for every frequency band of the audio sent to the ear from the audio source 12.

Differently from current systems, in which, as has been described in more detail above, the calibration is of the hybrid type and the velocity signal is determined for single frequencies, in the proposed system the calibration of the detector occurs only once before being assembled in the box-like body, with a pure, wide-band acoustic method.

It should also be noted that the direct detection, at a same point, of both pressure and velocity signals enables a more precise calculation of the acoustic immittance and the determination of the energy absorbance.

The combination of measurements of immittance and of absorbance of the audio make it possible to improve the investigation and possible detection of diseases of the tympanic membrane in the middle ear.

The box-like body 11, precisely for the set audiometric purposes can in practice be provided so as to reduce the residual volume of air contained in the box-like body to the possible minimum. Such box-like body, in fact, acts only as a support for the audio source 12 and detector 13 elements, and its extension can be reduced to the minimum necessary for the mechanical containment of the devices 12, 13, in that the volume of air of interest for the measurement of the acoustic immittance in the audiometric field is the one contained in the auditory canal and in the tympanic cavity.

This construction enables the reduction of residual volumes of air, which otherwise would render the apparatus ineffective for the measurement of any non-linear effects owing to what are called otoacoustic emissions, i.e. acoustic signals generated by outer ciliated cells, as an expression of active non-linear mechanisms inside the cochlea.

In practice it has been found that the construction fully achieves the intended aim and objects by providing an apparatus for clinical-audiometric investigation which measures acoustic energy values reliably and precisely, thus constituting a valid tool for the diagnosis of diseases of the tympanic membrane.

Moreover the storing of the data acquired makes it possible to also use the apparatus for the indirect validation of other diagnostic techniques, such as analysis of acoustic otoemissions, both those artificially stimulated with adapted signals, and those recorded spontaneously.

Another advantage of the apparatus is the ability to reuse some intermediate data, which are conveniently stored during the process of measurement, for the acoustic optimization of the response of acoustic prostheses or in acoustic tests of test volumes or of environments or of particular manufactured items other than the auditory canal of the human ear.

The construction, thus conceived, is susceptible of numerous modifications and variations. Moreover, all the details may be substituted by other, technically equivalent elements.

In practice the materials employed, provided they are compatible with the specific use, and the contingent dimensions and shapes, may be any according to requirements and to the state of the art.

Where the technical features mentioned in any claim are followed by reference numerals and/or signs, those reference numerals and/or signs have been included for the sole purpose of increasing the intelligibility of the claims and accordingly, such reference numerals and/or signs do not have any limiting effect on the interpretation of each element identified by way of example by such reference numerals and/or signs.

## Claims

1. An apparatus (10) for clinical-audiometric investigation, provided with a box-like body (11) that contains at least one audio source (12) of an acoustic signal (A) and at least one detector (13) of the return signal (B) from an external auditory canal (14), said box-like body (11) being associated with at least one processing and control device (15), said return signal detector (13) comprising a velocity sensor (16) and a pressure sensor (17), which measure the acoustic velocity and pressure signals at the same point, wherein said box-like body (11) is substantially cylindrical and hollow and has said audio source (12) arranged therein at one end and terminates at the other end with a frustum-shaped adapter (20) for coupling to the external auditory canal (14), **characterised in that** said signal detector (13) , including the velocity sensor (16) and pressure sensor (17), is arranged inside the box-like body (11) in an intermediate position between said audio source (12) and the said frustum-shaped adapter for coupling to the external auditory canal (14) from which said return signal (B) arrives.

2. The apparatus according to claim 1, **characterized in that** said processing and control device (15) comprises a first calculation section (18) and a second calculation section (19), which are assigned respectively to calculating the acoustic immittance and the energy analysis of the return signal (B).

3. The apparatus according to claim 1, **characterized in that** said velocity sensor (16) is constituted by an acoustic anemometer.

4. The apparatus according to claim 1, **characterized in that** said pressure sensor (17) is constituted by a microphone.

5. The apparatus according to claim 1, **characterized in that** it comprises an amplifier (21) of the signal (C) in output from said processing and control device (15) and directed toward said audio source (12).

6. The apparatus according to claim 1, **characterized in that** said processing and control device (15) is provided with a digital-to-analog converter (22) of the output signal (C).

7. The apparatus according to one or more of the preceding claims, **characterized in that** said processing and control device (15) is provided with an analog-to-digital converter (23) of a first input signal (D) that arrives from said velocity sensor (16) and of a second input signal (E) that arrives from said pressure sensor (17).

## Patentansprüche

1. Eine Vorrichtung (10) zur klinisch-audiometrischen Untersuchung, ausgestattet mit einem kastenähnlichen Körper (11), der mindestens eine Audioquelle (12) für ein akustisches Signal (A) und mindestens einen Detektor (13) für das Antwortsignal (B) von einem externen akustischen Kanal (14) enthält, wobei der kastenähnliche Körper (11) mit mindestens einer Verarbeitungs- und Steuervorrichtung (15) verbunden ist, wobei der Antwortsignaldetektor (13) einen Geschwindigkeitssensor (16) und einen Drucksensor (17) umfasst, welcher die Schallschnelle und die Drucksignale an demselben Punkt messen, wobei der kastenähnliche Körper (11) im Wesentlichen zylindrisch und hohl ist und die Audioquelle (12) darin an einem Ende angeordnet ist und am anderen Ende in einem kegelstumpfförmigen Adapter (20) zur Kopplung mit dem externen akustischen Kanal (14) endet, **dadurch gekennzeichnet, dass** der Signaldetektor (13) einschließlich des Geschwindigkeitssensors (16) und des Drucksensors (17) im kastenähnlichen Körper (11) in einer intermediären Position zwischen der Audioquelle (12) und dem kegelstumpfförmigen Adapter zur Kopplung mit dem externen akustischen Kanal (14) angeordnet ist von welchem das Antwortsignal (B) kommt.

2. Die Vorrichtung gemäß Anspruch 1, **dadurch gekennzeichnet, dass** die Verarbeitungs- und Steuervorrichtung (15) einen ersten Berechnungsabschnitt (18) und einen zweiten Berechnungsabschnitt (19) umfasst, die zur Berechnung der akustischen Imittanz bzw. der Energieanalyse des Antwortsignals (B) bestimmt sind.

3. Die Vorrichtung gemäß Anspruch 1, **dadurch gekennzeichnet, dass** der Geschwindigkeitssensor (16) aus einem akustischen Anemometer besteht.

4. Die Vorrichtung gemäß Anspruch 1, **dadurch gekennzeichnet, dass** der Drucksensor (17) aus einem Mikrofon besteht.

5. Die Vorrichtung gemäß Anspruch 1, **dadurch gekennzeichnet, dass** sie einen Verstäker (21) für das Signal (C) umfasst, dass von der Verarbeitungs- und Steuervorrichtung (15) ausgegeben und zu der Audioquelle (12) geleitet wird.

6. Die Vorrichtung gemäß Anspruch 1, **dadurch gekennzeichnet, dass** die Verarbeitungs- und Steuervorrichtung (15) mit einem Digital/Analog-Wandler (22) für das Ausgangssignal (C) ausgestattet ist.

7. Die Vorrichtung gemäß einem oder mehreren der obigen Ansprüche, **dadurch gekennzeichnet, dass** die Verarbeitungs- und Steuervorrichtung (15) mit einem Analog/Digital-Wandler (23) für ein erstes Eingangssignal (D), das von dem Geschwindigkeitssensor (16) kommt und für ein zweites Eingangssignal (E) ausgestattet ist, das von dem Drucksensor (17) kommt.

## Revendications

1. Appareil (10) pour examen clinique audiométrique, muni d'un corps en forme de boîte (11) qui contient au moins une source audio (12) d'un signal acoustique (A) et au moins un détecteur (13) du signal de retour (B) venant d'un canal auditif externe (14), ledit corps en forme de boîte (11) étant associé à au moins un dispositif de traitement et de commande (15), ledit détecteur (13) de signal de retour comprenant un capteur de vitesse (16) et un capteur de pression (17), qui mesurent la vitesse acoustique et les signaux de pression au même point, où
ledit corps en forme de boîte (11) est sensiblement cylindrique et creux et comporte ladite source audio (12) disposée dans celui-ci au niveau d'une extrémité et se termine à l'autre extrémité par un adaptateur en forme de tronc (20) pour s'accoupler au canal auditif externe (14),
**caractérisé en ce que** ledit détecteur de signal (13), comprenant le capteur de vitesse (16) et le capteur de pression (17), est disposé à l'intérieur du corps en forme de boîte (11) dans une position intermédiaire entre ladite source audio (12) et ledit adaptateur en forme de tronc pour s'accoupler au canal auditif externe (14) d'où arrive ledit signal de retour (B).

2. Appareil selon la revendication 1, **caractérisé en ce que** ledit dispositif de traitement et de commande (15) comprend une première section de calcul (18) et une seconde section de calcul (19), qui sont assignées respectivement au calcul de l'immittance acoustique et de l'analyse d'énergie du signal de retour (B).

3. Appareil selon la revendication 1, **caractérisé en ce que** ledit capteur de vitesse (16) est constitué par un anémomètre acoustique.

4. Appareil selon la revendication 1, **caractérisé en ce que** ledit capteur de pression (17) est constitué par un microphone.

5. Appareil selon la revendication 1, **caractérisé en ce qu'**il comprend un amplificateur (21) du signal (C) en sortie dudit dispositif de traitement et de commande (15) et dirigé vers ladite source audio (12).

6. Appareil selon la revendication 1, **caractérisé en ce que** ledit dispositif de traitement et de commande (15) est muni d'un convertisseur numérique-analogique (22) du signal de sortie (C).

7. Appareil selon l'une ou plusieurs des revendications qui précèdent, **caractérisé en ce que** ledit dispositif de traitement et de commande (15) est muni d'un convertisseur analogique-numérique (23) d'un premier signal d'entrée (D) qui arrive dudit capteur de vitesse (16) et d'un second signal d'entrée (E) qui arrive dudit capteur de pression (17).
